(19) Europäisches Patentamt
European Patent Office
Office européen des brevets

(11) Publication number: 0 353 350 B1

(12) # EUROPEAN PATENT SPECIFICATION

(45) Date of publication of patent specification: 17.02.93

(51) Int. Cl.5: C07C 227/10, C07C 229/16

(21) Application number: 88306435.4

(22) Date of filing: 13.07.88

(54) **A process for preparing N-substituted amino acid esters.**

(43) Date of publication of application:
07.02.90 Bulletin 90/06

(45) Publication of the grant of the patent:
17.02.93 Bulletin 93/07

(84) Designated Contracting States:
CH DE FR GB LI NL

(56) References cited:
EP-A- 0 096 157
EP-A- 0 117 448
US-A- 4 542 234

(73) Proprietor: DAICEL CHEMICAL INDUSTRIES, LTD.
No. 1-Banchi, Teppo-cho
Sakai-shi Osaka-fu 590(JP)

Proprietor: Tanabe Seiyaku Co., Ltd.
No. 21 Dosho-machi 3-chome Higashi-ku
Osaka-shi Osaka-fu(JP)

(72) Inventor: Takase, Ichirou
940, Shinzaike Aboshi-ku
Himeji-shi Hyogo-ken(JP)
Inventor: Yamasaki, Noritsugu
500, Kamiyobe Yobe-ku
Himeji-shi Hyogo-ken(JP)
Inventor: Sato, Kazuo
500, Kamiyobe Yobe-ku
Himeji-shi Hyogo-ken(JP)
Inventor: Goto, Yukihisa
1903-3, Okihama Aboshi-ku
Himeji-shi Hyogo-ken(JP)

(74) Representative: Jump, Timothy John Simon et al
Venner Shipley & Co. 20 Little Britain
London EC1A 7DH (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for preparing N-substituted amino acid esters. More particularly, it relates to an asymmetric synthetic method of preparing N-substituted α-amino acid esters from α-amino acid esters and α-substituted carboxylic acid esters.

Some N-substituted amino acid esters are useful as intermediates in the synthesis of various amino acid derivatives which show inhibitory action on angiotension-transformation enzyme. Said derivatives, therefore, can be used as antidepressants and are as useful as various physiologically active substances such as amavadin, histopine and octopine of natural origins.

(S,S)-N-substituted amino acid esters have been prepared e.g., by reacting an (S)-α-amino acid ester with an equimolar amount of an α-halocarboxylic acid ester in the presence of sodium carbonate and in an organic solvent such as dimethyl formamide. The yields however were not satisfactory, in the order of 46%, based upon theoretical yield of (S,S)-diasteroisomer (see US 4,542,234). Similar methods carried out in organic solvents are also known from US 4,344,949; US 4,596,791 and Japanese Unexamined Patent Publication No. Sho 60(1985)-13715.

N-Substituted amino acid esters also have been prepared by the reaction of amino acid esters with trifluoromethyl-sulfonyloxy substituted carboxylic acid esters in the presence of triethylamine and in an organic solvent, such as methylene chloride, with high yield (see DE 3,303,344, Japanese Unexamined Patent Publication No. Sho 59(1984)-172442).

It has been also reported that in the preparation of N-substituted amino acid esters from amino acid esters and α-halo carboxylic esters, α-tosyloxy carboxylic acid esters or α-mesyloxy carboxylic acid esters, a silver ion catalyst was required to achieve high yield (see US 4,350,704).

In such known methods, all the reactions are conducted in homogeneous system using organic solvents and do not afford sufficient yield, unless a carboxylic acid ester having a very active trifluorosulfonyloxy group, or a silver ion catalyst is used. Carboxylic acid esters having trifluorosulfonyloxy groups must be handled with care, because they are unstable and show tearing property. Accordingly, it is desired to develop a method for the preparation of N-substituted amino acid esters in high yield which can be easily operated and which can be carried out without resorting to severe reaction conditions.

Thus, the inventors have found an efficient asymmetric synthetic method of preparing N-substituted amino acid esters which is conducted in the absence of solvent and affords a good yield.

This invention provides a process for preparing an N-substituted amino acid ester having the formula (I):

$$R_1O_2C - \overset{\displaystyle R_2}{\overset{|}{CH}} - NH - \overset{\displaystyle R_3}{\overset{|}{CH}} - CO_2R_4 \qquad (I)$$

, wherein $R_1$ and $R_4$ are the same or different, each being an alkyl, aralkyl, cycloalkyl or aryl group and, $R_2$ and $R_3$ are the same or different, each being an alkyl, aralkyl, aryl, heterocyclo-alkyl, aminoalkyl or guanidylalkyl group which process comprises heating and reacting an α-amino acid ester of the formula (II):

$$R_1O_2C - \overset{\displaystyle R_2}{\overset{|}{CH}} - NH_2 \qquad (II)$$

, wherein $R_1$ and $R_2$ have the same meaning as in formula (I), with a α-substituted carboxylic acid ester of the formula (III):

$$X - \overset{\displaystyle R_3}{\overset{|}{CH}} - CO_2R_4 \qquad (III)$$

, wherein X is a leaving group and $R_3$ and $R_4$ have the same meaning as in formula (I), in the absence of solvent and thereafter isolating the resultant N-substituted amino acid ester of formula (I).

Optionally the reaction may be carried out in the presence of a base. Preferably, said alkyl groups are straight chain or branched chain alkyl having one to six carbon atoms such as methyl, ethyl, propyl, isopropyl, n-butyl, iso-butyl, sec-butyl or tert-butyl. Examples of said aralkyl groups are benzyl, $\alpha$-phenethyl, $\beta$-phenethyl, 4-hydroxybenzyl or 3,4-dihydroxybenzyl. Examples of said cycloalkyl groups are cyclopentyl, cyclohexyl or cycloheptyl. Examples of said aryl groups are phenyl or phenyl substituted by an alkyl, a halogen, nitro or hydroxy.

The heterocyclo-substituted alkyl group may be imidazolylmethyl, the aminoalkyl group may be 4-amino-butylbutyl, and the guanidylalkyl may be guanidylmethyl or guanidylethyl.

The leaving group X of formula (III) may be a halogen atom such as chlorine, bromine or iodine; art aliphatic sulfonyloxy group such as methane sulfonyloxy, ethane sulfonyloxy or butane sulfonyloxy, an aromatic sulfonyloxy group such as benzene sulfonyloxy, p-toluene sulfonyloxy, p-bromobenzene sulfonyloxy or p-nitrobenzene sulfonyloxy; or a halo sulfonyloxy such as chlorosulfonyloxy.

The N-substituted amino acid ester of formula (I) includes (S,S)-and (R,R)-configurations as absolute structure thereof. The starting materials, i.e., the compound of the formula (II), as well as the compound of the formula (III) are selected from those having (S) or (R)-configuration in order to give a product of the desired structure. Especially, in accordance with the process of this invention, an (S,S)-compound of the formula (I) can be obtained in a good yield, when an (R)-compound of the formula (II) and an (S)-compound of the formula (III) are used. Similarly, an (R,S)-compound, an (S,R)-compound or an (R,R)-compound of the formula (I) may be obtained from reacting an (R)-compound (II) with an (R) -compound (III), an (S)-compound (II) with an (S)-compound (III) or, an R) -compound (II) with an (R) -compound (III), respectively.

The starting materials, the compounds of the formula (II) or (III), are partially known and also can be easily prepared from known compounds using conventional methods. For example, the compound (II) can be prepared by esterifying, with an appropriate alcohol, an amino acid such as alanine, phenylalanine, tyrosine, homophenylalanine, valine, lysine, arginine, histidine, phenylglycine, 4-hydroxyphenyl glycine or dopa-(3-(3,4-dihydroxyphenyl)alanine). Also, the compound (III) can be prepared e.g., by diazoating an amino acid followed by halogenating, hydrating or sulfonating, or by asymmetrically reducing a -keto acid, followed by halogenation or sulfonation.

The amino acids as mentioned above are preferrably used in optically active forms having (R) or (S)-configuration but when the racemates are used, the products may then be resolved.

In the process of this invention, the compound (II) is reacted with the compound (III) in the absence of solvent.

The wording "in the absence of solvent" means that any solvent is not positively or intentionally added or employed. However, the reaction system may contain an organic solvent included with a starting material, which solvent comes from the manufacturing process of the starting material and comprises about 5 or like % of the starting material.

It has been found that the yield of the reaction in accordance with this invention which is conducted in the absence of a solvent, is significantly improved in comparison with that of a reaction in the presence of an organic solvent, although the yield may be influenced by the nature and ratios of the starting materials and so forth.

It is preferred to use either one of the compound (II) or the compound (III) in an excess amount, e.g., 1.1 or more mols of one compound with one mol of the other. It is especially preferred to use two or more molar amounts of the compound (II) to one mol of the compound (III). In such a case, the reaction may be conducted without addition of a base to neutralise any acidic substance formed by the reaction, since the excess of compound (II) existing will act as a base. When the compound (II) is used in excess, it is easily recovered after the reaction and may be used in another batch of the reaction. However, it is not generally recommended from the industrial viewpoint to use four or more molar amounts of the compound (III), because it is rather expensive.

The reaction of this invention may be carried out in the presence of a base. The base is usually used in an amount nearly equal to the amount of either compound (II) or (III), whichever is in the minority. However, when the base is potassium or sodium carbonate, which may make the reaction system a heterogeneous one, it is desirably used in an amount between 1.0 and 2.0 times that of either compound (II) or (III), whichever is in the minority.

Examples of suitable bases include organic bases such as triethylamine, trioctylamine, pyridine, N,N-dimethylaminopyridine and diazabicycloundecene, or inorganic bases such as sodium carbonate, potassium carbonate, lithium carbonate and magnesium carbonate as well as their corresponding hydrogen carbonates. It is preferable to use sodium carbonate or potassium carbonate, although it is insoluble in any of

the compounds of the formulae (II) and (III) because it provides a higher yield of the object compound (I) than the organic bases.

The reaction is generally carried out between a temperature slightly elevated from room temperature and a temperature just below that which would cause decomposition of the raw materials, preferably at 50 °C - 130 °C, more preferably at 70 °C - 110 °C. The reaction will be generally completed in a period between several hours and a day. Furthermore, the reaction mixture is preferably stirred whilst the reaction is in progress.

After completing the reaction, the N-substituted amino acid ester of formula (I) may be isolated e.g., by adding water and ethyl ether to the reaction mixture, collecting the ether layer, removing the solvent, dissolving the oily residue in a mixture of methylene chloride and ethyl acetate (20 : 1) and subjecting the solution to silica gel-chromatography. Alternative conventional methods may be utilized to isolate the object compound.

This invention is further illustrated by examples.

## Example 1

Preparation of benzyl N-((1S)-1-ethoxycarbonyl-3-phenylpropyl)-L-alanate (1-a)

Ethyl (S)-homophenylalanate (0.415 g, 2.00 mmol), benzyl (R)-α-toluenesulfonyloxypropionate (0.661 g, 2.00 mmol) and potassium carbonate (0.207 g, 1.50 mmol) were added to a two-necked flask equipped with a condenser and a thermometer, and stirred for 6 hours at 70 °C. After completing the reaction, the mixture was gradually cooled to 60 °C, and toluene (10 ml) was added thereto in order to prevent hardening. The mixture was cooled to room temperature and water was added to the mixture to dissolve any soluble salts. Then, the mixture was extracted with toluene twice. The combined toluene layers were dried over magnesium sulfate, filtered and concentrated under reduced pressure. The obtained pale yellow oil was subjected to column-chromatography on silica gel, eluting with methylene chloride-ethyl acetate (20 : 1 (v/v)), to give 0.608 g (1.65 mmol, 82,3%) of the title compound (1-a). Its physical properties are as follows.

$[\alpha]^{25}_{D}$ = -18.4° (c = 1, $CHCl_3$)

| | |
|---|---|
| $^1$H-NMR(δ,ppm) : | 1.23(t,J = 7Hz,3H), 1.31(d,J = 7Hz,3H), 1.80(br,1H) 1.70-2.19(m,2H) 2.55-2.82(m,2H), 3.20-3.46(m,2H) 4.12 (q,J = 7Hz,2H), 5.08(s,2H) 7.12 (s,5H), 7.26 (s,5H) |
| IR($cm^{-1}$) : | 1740 (C = 0) |
| MS(m/z) : | 369($M^+$), 296($M^+$-$CO_2$Et), 234 ($M^+$ -$CO_2CH_2$Ph) |

(S,S)-compound : (R,R)-compound : diastereomer (S,R + R,S) 98.4 : 0 : 1.6 (by HPLC using column for optical resolution)

## Examples 2 - 15

In a similar way to Example 1, ethyl(S)-homophenylalanate [II; $R_1$ = Et, $R_2$ = $Ph(CH_2)_2$] and benzyl (R)-α-toluenesulfonyloxypropionate [III, $R_3$ = Me, $R_4$ = $Ph(CH_2)_2$] in the presence or absence of a base were reacted under the conditions mentioned in Table 1. The reaction mixture was treated in a similar way to Example 1 to give benzyl N-[(1S)-1-ethoxycarbonyl-3-phenylpropyl]-L-alanate (I-a).

Table 1

| Examle No. | Ratio in Mol. of the raw materials | | | Reaction Temp. (°C) | Reaction Time (hr.) | Yield (%) on (S,S)-isomer | Ratio of isomers | Optical* rotation (°) |
|---|---|---|---|---|---|---|---|---|
| | (II) | (III) | base | | | | (S,S) : (R,R) : diasteromer | |
| 2 | 6 | 2 | — | 90 | 3.0 | 87.7 | 98.8 : 0 : 1.2 | -18.9 |
| 3 | 6 | 2 | — | 90 | 6.0 | 89.2 | 98.8 : 0 : 1.2 | -18.9 |
| 4 | 4 | 2 | — | 90 | 6.0 | 87.9 | 98.7 : 0 : 1.3 | -18.5 |
| 5 | 2.2 | 2 | $K_2CO_3$ 1.0 | 80 | 12 | 82.5 | 97.8 : 0 : 2.2 | -17.8 |
| 6 | 2.2 | 2 | $K_2CO_3$ 1.0 | 60 | 24 | 79.5 | 98.9 : 0 : 1.1 | -17.0 |
| 7 | 2 | 2 | $K_2CO_3$ 1.0 | 90 | 6.0 | 69.8 | 98.9 : 0 : 1.2 | -18.4 |
| 8 | 2 | 2 | $K_2CO_3$ 1.0 | 80 | 12 | 77.5 | 97.8 : 0 : 2.2 | -17.8 |
| 9 | 2 | 2 | $K_2CO_3$ 1.0 | 60 | 24 | 61.2 | 98.9 : 0 : 1.1 | -17.0 |
| 10 | 2 | 2 | $K_2CO_3$ 1.5 | 80 | 6.0 | 64.9 | 99.0 : 0 : 1.0 | -18.4 |
| 11 | 2 | 3 | $K_2CO_3$ 1.5 | 90 | 3.0 | 81.4 | 98.2 : 0 : 1.8 | -18.4 |
| 12 | 2 | 4 | $K_2CO_3$ 1.5 | 90 | 3.0 | 86.5 | 98.9 : 0 : 1.1 | -18.7 |
| 13 | 2 | 6 | $K_2CO_3$ 1.5 | 90 | 3.0 | 88.2 | 99.0 : 0 : 1.0 | -18.6 |
| 14 | 2 | 2 | MgO 1.0 | 90 | 6.0 | 70.7 | 95.7 : 0 : 4.3 | -18.1 |
| 15 | 2 | 2 | $Et_3N$ 3.0 | 90 | 3.0 | 90.0 | 90.3 : 0.55 : 9.2 | -16.1 |

* $[\alpha]_D^{25}$ (C=1, $CHCl_3$)

## Examples 16 - 25

Instead of ethyl (S)-homophenylalanate and benzyl (R)-α-toluenesulfonyloxypropionate used in Example 1, other (S)-amino acid esters (II) and (R)-carboxylic acid esters (III) were reacted and subjected to after-treatment in the same way as Example 1. The reactants and the conditions of these reactions are shown in Table 2.

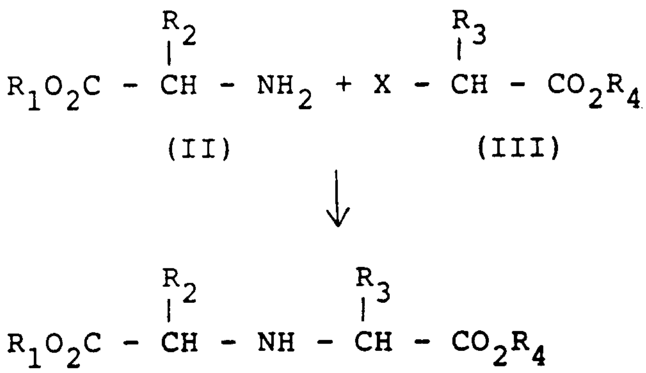

(II) (III)

(I)

Table 2

| Example No. | (II) R1 | (II) R2 | (III) R3 | (III) R4 | (III) X | Ratio in Mol. (II) | Ratio in Mol. (III) | Ratio in Mol. base | (I) | yield (%) | Optical rotation $[\alpha]_D$ (°) |
|---|---|---|---|---|---|---|---|---|---|---|---|
| 16 | Et | Me | Me | Me | OTs | 9 | 3 | — | I-b | 89.3 | -42.4 |
| 17 | Et | Me | Me | Me | OTs | 2 | 4 | $K_2CO_3$ 1.5 | I-b | 85.4 | -42.0 |
| 18 | Et | $PhCH_2$ | Me | Me | OTs | 2 | 4 | $K_2CO_3$ 1.5 | I-c | 87.1 | |
| 19 | Et | $Ph(CH_2)_2$ | Me | $PhCH_2$ | OMs | 9 | 3 | — | I-a | 89.6 | -18.9 |
| 20 | Et | $Ph(CH_2)_2$ | Me | $PhCH_2$ | OMs | 2 | 3 | $K_2CO_3$ 1.5 | I-a | 83.7 | -18.4 |
| 21 | Et | $Ph(CH_2)_2$ | Me | Me | OTs | 10 | 10 | $K_2CO_3$ 7.5 | I-d | 74.9 | -14.9 |
| 22 | Et | $Ph(CH_2)_2$ | Me | Et | OTs | 10 | 10 | $K_2CO_3$ 7.5 | I-e | 80.9 | -12.7 |
| 23 | $PhCH_2$ | $Ph(CH_2)_2$ | Me | $PhCH_2$ | OTs | 6.4 | 6.4 | $K_2CO_3$ 4.8 | I-f | 74.7 | |
| 24 | $PhCH_2$ | $Ph(CH_2)_2$ | Me | Me | OTs | 7.4 | 8.1 | $K_2CO_3$ 11.2 | I-g | 64.8 | |
| 25 | $PhCH_2$ | $Ph(CH_2)_2$ | Me | Et | OTs | 5 | 5 | $K_2CO_3$ 3.75 | I-h | 71.6 | -22.2 |

The physical properties of the compounds obtained in Examples 16 - 25 are as follows:

[I-b] methyl N-[(1S)-1-ethoxycarbonylethyl]-L-alanate
$C_9H_{17}NO_4$ = 203.24
$[\alpha]^{28}_D$ = -42.4° ($CHCl_3$, c=0.86)

$^1$H-NMR(δ,ppm) : 1.28(t,J=7Hz,3H), 1.33(d,J=7Hz,3H), 1.34(d,J=7Hz,3H), 2.23(s,1H), 3.40-(q,J=7Hz,1H), 3.42(q,J=7Hz,1H), 3.73(s,3H), 4.18(q,J=7Hz,2H)

[I-c] methyl N-[(1S)-1-ethoxycarbonyl-2-phenylethyl]-L-alanate
$C_{15}H_{21}NO_4$ = 279.34

$^1$H-NMR(δ,ppm) : 1.17(t,J=7Hz,3H), 1.27(d,J=7Hz,3H), 1.93(s,1H), 2.87-2.95(m,2H),3.20-3.47(m,2H), 3.60(s,3H), 4.10 (q,J=7Hz,2H), 7.17(s,5H)

[I-d] methyl N-[(1S)-1-ethoxycarbonyl-3-phenylpropyl]-L-alanate
$C_{16}H_{23}NO_4$ = 293.36
$[\alpha]^{31}_D$ = -14.9° (c=1.0, $CHCl_3$)

$^1$H-NMR(δ,ppm) : 1.27(t,7Hz,3H), 1.32(d,J=7Hz,3H), 1.83(s,1H), 1.70-2.13(m,2H), 2.58-2.83(m,2H), 3.30(t,J=7Hz,1H), 3.35(q,J=7Hz,1H), 3.67(s,3H), 4.12(q,J=7Hz,2H), 7.15(s,5H)

[I-e] ethyl N-[(1S)-1-ethoxycarbonyl-3-phenylpropyl]-L-alanate
$C_{17}H_{25}NO_4$ = 307.39
$[\alpha]^{31}_D$ = -12.7° (c=1.2, $CHCl_3$)

$^1$H-NMR(δ,ppm) : 1.27(t,J=7Hz,3H), 1.30(t,J=7Hz,3H), 1.35(d,J=7Hz,3H), 1.83(s,1H), 1.75-2.10 (m,2H), 2.60-2.85(m,2H), 3.30 (t,J=7Hz,1H), 3.33(q,J=7Hz,1H) 4.13(q,J=7Hz,2H), 4.17(q,J=7Hz,2H), 7.20(s,5H)

[I-f] benzyl N-[(1S)-1-benzyloxycarbonyl-3-phenylpropyl]-L-alanate
$C_{27}H_{29}NO_4$ = 431.53

$^1$H-NMR(δ,ppm) : 1.30(d,J=7Hz,3H), 1.77(s,1H), 1.67-2.00(m,2H), 2.50-2.77 (m,2H), 3.37-(t,J=7Hz,1H), 3.40(q,J=7Hz,1H) I 5.05(s,4H), 7.07(s,5H), 7.23(s,10H)

[I-g] methyl N-[(1S)-1-benzyloxycarbonyl-3-phenylpropyl]-L-alanate
$C_{21}H_{25}NO_4$ = 355.43

$^1$H-NMR(δ,ppm) : 1.32(d,J=7Hz,3H), 1.87(s,1H), 1.73-2.10(m,2H), 2.53-2.80(m,2H), 3.36(q,J=7Hz,1H). 3.37(t,J=7Hz,1H), 3.67 (s,3H), 5.13(s,2H), 7.15(s,5H), 7.30(s,5H)

[I-h] ethyl N-[(1S)-1-benzyloxycarbonyl-3-phenylpropyl]-L-alanate
$C_{22}H_{27}NO_4$ = 369.46
$[\alpha]^{24}_D$ = -22.24° (c=1.0, $CHCl_3$)

$^1$N-NMR(δ,ppm) : 1.23(t,J=7Hz,3H), 1.28(d,J=7Hz,3H), 1.80 (s,1H) 1.73-2.10(m,2H), 2.53-2.79 (m,2H), 3.33 (q,J=7Hz, 1H), 3.39 (t,J=7Hz,1H), 4.10(q,J=7Hz,2H), 5.07 (s,2H) 7.07 (s,5H), 7.23(s,5H)

Comparative Examples 1 - 3 (US 4,542,234)

Ethyl (S)-homophenylalanine (I′) and benzyl (R) -α-toluenesulfonyloxypropionate (II′) in the presence or absence of potassium carbonate were added to acetonitrile (15 ml, bp 82°C), and refluxed for a predetermined time, under stirring. After completing the reaction, the mixture was concentrated under reduced pressure and water (10ml) was added to the oily residue. Then the mixture was extracted with toluene (10 ml). The organic layer was concentrated under reduced pressure to remove toluene. The oily residue was subjected to silica gel column chromatography to give the compound of N-[(1S) -1-ethoxycarbonyl-3-phenylpropyl]-L-alanine. The reaction conditions are shown in Table 3.

Table 3

| Reference Example No. | Ratio in Mol. | | | Reaction Time (hr.) | Yield of (S,S) isomer | Ratio of Optical Isomer (S:S) : (R:R) : diastereomer |
|---|---|---|---|---|---|---|
| | (I') | (II') | $K_2CO_3$ | | | |
| 1 | 2.0 | 3.0 | 1.0 | 6 | 30.9 | 99.0 : 0 : 1.0 |
| 2 | 4 | 2 | — | 12 | 42.5 | 99.0 : 0 : 1.0 |
| 3 | 8 | 2 | — | 12 | 69.0 | 99.0 : 0 : 1.0 |

## Claims

1. A process for preparing an N-substituted amino acid ester having the formula (I):

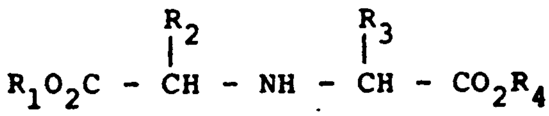

(I)

, wherein $R_1$ and $R_4$ are the same or different, each being an alkyl, aralkyl, cycloalkyl or aryl group and, $R_2$ and $R_3$ are the same or different, each being an alkyl, aralkyl, aryl, heterocyclo-alkyl, aminoalkyl or guanidylalkyl group, which process comprises heating and reacting an $\alpha$-amino acid ester of the formula (II):

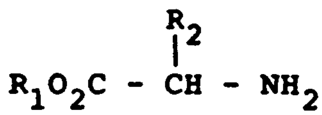

(II)

, where $R_1$ and $R_2$ have the same meanings as in formula (I), with an $\alpha$-substituted carboxylic acid ester of the formula (III):

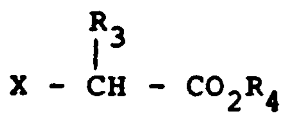

(III)

, wherein X is a leaving group and $R_3$ and $R_4$ have the same meanings as in formula (I), and thereafter isolating the resultant N-substituted amino acid of formula (I) characterised in that the reaction between the $\alpha$-amino acid ester of formula (II) and the $\alpha$-substituted carboxylic acid ester of formula (III) is carried out in the absence of solvent.

2. A process as claimed in claim 1 characterised in that the reaction is carried out in the presence of a base.

3. A process as claimed in claim 1 or claim 2 characterised in that the reaction temperature is in the range of 50 °C to 130 °C.

4. A process as claimed in claim 3 characterised in that the reaction temperature is in the range of 70 °C to 110 °C.

5. A process as claimed in claim 2 characterised in that the base is used in an equimolar or greater quantity as either the compound of formula (II) or the compound of formula (III).

6. A process as claimed in claim 5 characterised in that the compound (II) is used in two to four molar amounts to one mol of the compound (III).

7. A process as claimed in claim 2 characterised in that the base is used in an equivalent or greater amount to the compound selected from the compound of formula (II) and the compound of formula (III) which is used in a lesser amount.

8. A process as claimed in claims 2-7 characterised in that the base is potassium carbonate.

9. A process as claimed in claim 1 characterised by being adapted to prepare (S,S)-compound (I) from (S)-compound (II) and (R)-compound (III).

**10.** A process as claimed in claim 1 characterised by being adapted to prepare (R,R)-compound (I) from (R)-compound (II) and (S)-compound (III).

**11.** A process as claimed in any of the preceeding claims characterised in that each of $R_1$ and $R_4$ is a straight or branched chain alkyl group having from 1 to 6 carbon atoms; a benzyl; $\alpha$-phenethyl; $\beta$-phenethyl; 4-hydroxbenzyl; 3,4-dihydroxybenzyl; cyclopentyl; cyclohexyl; cycloheptyl; phenyl; or an alkyl, halogen, nitro or hydroxy substituted phenyl group: and in that each of $R_2$ and $R_3$ is a straight or branched chain alkyl group having from 1 to 6 carbon atoms; a benzyl; $\alpha$-phenethyl; $\beta$-phenethyl; 4-hydroxybenzyl; 3,4-dihydroxybenzyl; phenyl; an alkyl, halogen, nitro or hydroxy substituted phenyl; imidazolylmethyl; 4-amino-butylbutyl; guanidylmethyl or; guanidylethyl group.

**Patentansprüche**

**1.** Verfahren zur Herstellung eines N-substituierten Aminosäureesters mit der Formel (I):

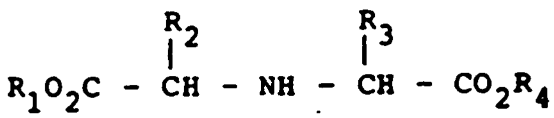

(I)

worin $R_1$ und $R_4$ gleich oder verschieden sind und jeweils für eine Alkyl-, Aralkyl-, Cycloalkyl- oder Arylgruppe stehen, und $R_2$ und $R_3$ gleich oder verschieden sind und jeweils für eine Alkyl-, Aralkyl-, Aryl-, Heterocycloalkyl-, Aminoalkyl- oder Guanidylalkylgruppe stehen, durch Erhitzen und Umsetzen eines -Aminosäureesters der Formel (II):

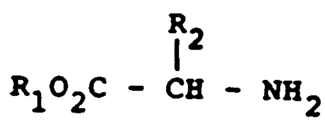

(II)

worin $R_1$ und $R_2$ die gleichen Bedeutungen, wie im Zusammenhang mit Formel (I) angegeben, haben, mit einem $\alpha$-substituierten Carbonsäureester der Formel (III):

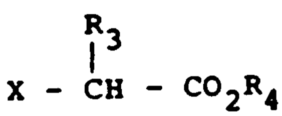

(III)

worin X eine Austrittsgruppe ist und $R_3$ und $R_4$ die gleichen Bedeutungen, wie im Zusammenhang mit Formel (I) angegeben, haben, und anschließendes Isolieren der resultierenden N-substituierten Amninosäure der Formel (I), dadurch **gekennzeichnet,** daß man die Reaktion zwischen dem $\alpha$-Aminosäureester der Formel (II) und dem $\alpha$-substituierten Carbonsäureester der Formel (III) in Abwesenheit von Lösungsmittel durchführt.

**2.** Verfahren nach Anspruch 1, dadurch **gekennzeichnet,** daß man die Reaktion in Gegenwart einer Base durchführt.

**3.** Verfahren nach Anspruch 1 oder 2, dadurch **gekennzeichnet,** daß die Reaktionstemperatur im Bereich von 50°C bis 130°C liegt.

**4.** Verfahren nach Anspruch 3, dadurch **gekennzeichnet,** daß die Reaktionstemperatur im Bereich von 70°C bis 110°C liegt.

**5.** Verfahren nach Anspruch 2, dadurch **gekennzeichnet,** daß man die Base in einer äquimolaren oder größeren Menge als entweder die Verbindung der Formel (II) oder die Verbindung der Formel (III)

einsetzt.

6. Verfahren nach Anspruch S, dadurch **gekennzeichnet,** daß man die Verbindung (II) in der zweibis vierfachen molaren Menge, bezogen auf 1 mol der Verbindung (III), einsetzt.

7. Verfahren nach Anspruch 2, dadurch **gekennzeichnet,** daß man die Base in einer gleichen oder größeren Menge als die Verbindung, ausgewählt aus der Verbindung der Formel (II) und der Verbindung der Formel (III), die in einer kleineren Menge eingesetzt wird, verwendet.

8. Verfahren nach den Ansprüchen 2 bis 7, dadurch **gekennzeichnet,** daß die Base Kaliumcarbonat ist.

9. Verfahren nach Anspruch 1, dadurch **gekennzeichnet,** daß es so ausgestaltet ist, daß die (S,S)-Verbindung (I) aus der (S)-Verbindung (II) und der (R)-Verbindung (III) hergestellt wird.

10. Verfahren nach Anspruch 1, dadurch **gekennzeichnet,** daß es so ausgestaltet ist, daß die (R,R)-Verbindung (I) aus der (R)-Verbindung (II) und der (S)-Verbindung (III) hergestellt wird.

11. Verfahren nach einem der vorstehenden Ansprüche, dadurch **gekennzeichnet,** daß jedes von $R_1$ und $R_4$ eine geradkettige oder verzweigtkettige Alkylgruppe mit 1 bis 6 Kohlenstoffatomen, eine Benzyl-, -Phenethyl-, β-Phenethyl-,4-Hydroxybenzyl-, 3,4-Dihydroxybenzyl-, Cyclopentyl-, Cyclohexyl-, Cycloheptyl-, Phenyl-, oder eine Alkyl-, Halogen-, Nitro- oder hydroxysubstituierte Phenylgruppe ist und daß jedes von $R_2$ und $R_3$ eine geradkettige oder verzweigtkettige Alkylgruppe mit 1 bis 6 Kohlenstoffatomen, eine Benzyl-, α-Phenethyl-, β-Phenethyl-, 4-Hydroxybenzyl, 3,4-Dihydroxybenzyl-, Phenyl-, eine Alkyl-, Halogen-, Nitro- oder hydroxy-substituierte Phenyl-, Imidazolylmethyl-, 4-Aminobutylbutyl-, Guanidylmethyl- oder Guanidylethylgruppe ist.

**Revendications**

1. Procédé pour préparer un ester d'aminoacide N-substitué ayant la formule (I) :

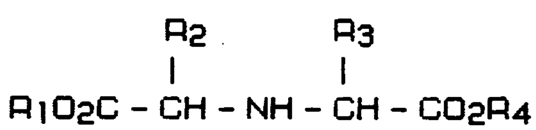

(I)

dans laquelle $R_1$ et $R_4$ sont identiques ou différents, et représentent chacun un groupe alkyle, aralkyle, cycloalkyle ou aryle, et $R_2$ et $R_3$ sont identiques ou différents et représentent chacun un groupe alkyle, aralkyle, aryle, hétérocycloalkyle, aminoalkyle ou guanidylalkyle, lequel procédé comprend le chauffage et la réaction d'un ester d'α-aminoacide de formule (II) :

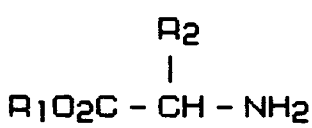

(II)

dans laquelle $R_1$ et $R_2$ sont tels que définis pour la formule (I), avec un ester d'acide carboxylique α-substitué de formule (III) :

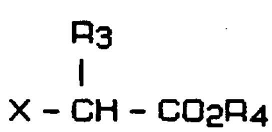

(III)

dans laquelle X est un groupe mobile et $R_3$ et $R_4$ sont tels que définis pour la formule (I), et ensuite l'isolation de l'aminoacide N-substitué résultant représenté par la formule (I), caractérisé en ce que la réaction entre l'ester d'α-aminoacide de formule (II) et un ester d'acide carboxylique α-substitué de

formule (III) est effectuée en l'absence de solvant.

**2.** Procédé suivant la revendication 1, caractérisé en ce que la réaction est effectuée en présence d'une base.

**3.** Procédé suivant les revendications 1 ou 2, caractérisé en ce que la température de réaction est de l'ordre de 50 à 130 ° C.

**4.** Procédé suivant la revendication 3, caractérisé en ce que la température de réaction est de l'ordre de 70 à 110 ° C.

**5.** Procédé suivant la revendication 2, caractérisé en ce que la base est utilisée en une quantité équimolaire ou supérieure à celle du composé de formule (II) ou du composé de formule (III).

**6.** Procédé suivant la revendication 5, caractérisé en ce que le composé (II) est utilisé à raison de deux à quatre quantités molaires par mole du composé (III).

**7.** Procédé suivant la revendication 2, caractérisé en ce que la base est utilisée en une quantité équivalente ou supérieure à celle du composé de formule (II) et du composé de formule (III) qui est utilisé en une moindre quantité.

**8.** Procédé suivant l'une quelconque des revendications 2 à 7, caractérisé en ce que la base est du carbonate de potassium.

**9.** Procédé suivant la revendication 1, caractérisé en ce qu'il est adapté pour préparer un composé (I) (S, S) à partir d'un composé (II) (S) et d'un composé (III) (R).

**10.** Procédé suivant la revendication 1, caractérisé en ce qu'il est adapté pour préparer un composé (I) (R, R) à partir d'un composé (II) (R) et d'un composé (III) (S).

**11.** Procédé suivent l'une quelconque des revendications précédentes, caractérisé en ce que chacun des $R_1$ et $R_4$ est un groupe alkyle à chaîne droite ou ramifiée ayant de 1 à 6 atomes de carbone, un groupe benzyle, $\alpha$-phénéthyle, $\beta$-phénéthyle, 4-hydroxybenzyle, 3,4-dihydroxybenzyle, cyclopentyle, cyclohexyle, cycloheptyle, phényle, ou groupe phényle substitué par un atome d'halogène, un groupe alkyle, nitro ou hydroxy; et que chacun des $R_2$ et $R_3$ est un groupe alkyle à chaîne droite ou ramifiée ayant de 1 à 6 atomes de carbone, un groupe benzyle, $\alpha$-phénéthyle, $\beta$-phénéthyle 4-hydroxybenzyle, 3,4-dihydroxybenzyle, phényle, ou phényle substitué par un atome d'halogène, un groupe alkyle, nitro ou hydroxy; imidazolylméthyle, 4-amino-butylbutyle, guanidylméthyle ou guanidyléthyle.